(19) **Europäisches Patentamt**
**European Patent Office**
**Office européen des brevets**

(11) **EP 2 163 899 A1**

(12) # EUROPEAN PATENT APPLICATION

(43) Date of publication:
**17.03.2010 Bulletin 2010/11**

(51) Int Cl.:
***G01N 33/68*** (2006.01)

(21) Application number: **08164089.8**

(22) Date of filing: **10.09.2008**

<table>
<tr><td>

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Koninklijke Philips Electronics N.V.**
**5621 BA Eindhoven (NL)**

</td><td>

(72) Inventor: **The designation of the inventor has not yet been filed**

(74) Representative: **Jungen, Carmen**
**Philips Intellectual Property &**
**Standards GmbH**
**Weißhausstraße 2**
**52066 Aachen (DE)**

</td></tr>
</table>

(54) **Sensitive fluorescence correlation measurements of soluble adhesion molecules**

(57) The present invention refers to a method for detecting soluble adhesion molecules, especially sVCAM-1, sICAM-1 and/or sE-selectin. The use of FCS or FCCS for detecting, analyzing or quantifying of soluble adhesion molecules being dissolved in a sample allows for assessing the risks or the progression of coronary artery disease.

EP 2 163 899 A1

## Description

FIELD OF THE INVENTION

The present invention refers to a method for detecting soluble adhesion molecules, especially sVCAM-1, sICAM-1 and/or sE-selectin.

BACKGROUND OF THE INVENTION

Coronary artery disease (CAD), also known as coronary heart disease (CHD) or coronary atherosclerosis, involves the progressive narrowing of the arteries that nourish the heart muscle. Often there are no symptoms, but if one or more of these arteries become severely narrowed, angina may develop during exercise, stress, or other times when the heart muscle is not getting enough blood. The narrowing is due to a buildup of fatty/proteinous plaque (atherosclerosis) along the artery walls. Coronary disease appears to be a lifelong process in some people, beginning at an early age and progressing slowly until the vessels become so occluded that the heart muscle no longer gets adequate nourishment.

It has been shown that endothelial and immune cell adhesion molecules play an important role in the development of coronary atherosclerosis and inflammatory disease. It has been found that specific soluble cell adhesion molecules and especially vascular cell adhesion molecule-1 (VCAM-1) and E-selectin are increased in patients with documented coronary artery disease (CAD). For example, patients with documented CAD exhibited significant elevation of the plasma levels of VCAM-1 and E-selectin as compared to subjects with normal coronary arteries. Furthermore, it has been demonstrated that major atherosclerotic risk factors appear during childhood and are also linked to changes in the endothelial wall consistent with CAD progression. As in adults, children with multiple CAD risk factors (such as obesity, diabetes and hypertension) demonstrate a more rapid progression of CAD.

During the progression of atherosclerosis, leukocytes can bind via integrins and other surface expressed proteins to intercellular adhesion molecule 1 (ICAM-1), vascular adhesion molecule 1 (VCAM-1), and selectins (E-, P- and L-) on the walls of endothelial cells. Facilitated by these protein-protein interactions, leukocytes infiltrate the endothelial wall, setting forth the cascade of inflammation responses in the arterial wall leading to the accumulation of (oxidized) lipid deposits and foam cells, the hallmarks of the atherosclerotic plaques. While the major medical complications associated with CAD present themselves later in adult life, the disease progression usually is initiated in childhood and adolescence, and it is this "decades long" progression of the disease that also makes the prediction of risk of suffering a coronary event so difficult. At the time of a myocardial infarction (MI), many interventional therapeutics are no longer useful, that is, during MI heart tissue is already dying.

The traditional immunobased assay (ELISA) utilized to examine sVCAM-1, sICAM-1 and sE-Selectin both require a lot of technical knowhow, specialized instrumentation. These ELISA type assays may be available in a central hospital laboratory, but the turn around times for such tests are not within the time scale of an average Emergency Room visit. Furthermore, there is a great variability between ELISA chips from various manufacturers, leading to inconsistent results between hospitals and patients, and hence industry standardization of such testing, is very challenging. Finally, while ELISAs can be very sensitive, the required sensitivity to resolve very minute changes in concentrations between an individual's samples is unlikely. Furthermore, ELISAs also require sophisticated chip-surface scanning instrumentation, and often surface artifacts add complexity to antigen-antibody interactions that are minimized in free solution.

In view of the foregoing observations, a non-invasive method that would allow for the reliable detection of high-risk individuals (usually in adolescence and early adult-hood) during the long presymptomatic phase of CAD progression, when changes to the endothelium are reversible, would greatly diminish the risk of major coronary events later in life, resulting in saved lives and diminished health care costs. There is a clear need to better stratify risk patients with CAD, prior to major coronary complications, such that adequate interventional therapies, such as surgery or drug treatment may prevent the further progression of the disease and prevent the damage of heart and other tissues. Furthermore, rapid and sensitive testing of blood based markers can aid greatly in the evaluation of therapy effectiveness.

SUMMARY OF THE INVENTION

It is an object of the present invention to provide a method for detecting soluble adhesion molecules, especially sVCAM-1, sICAM-1 and/or sE-selectin.

It is also an object of the present invention to provide a very sensitive method for detecting soluble adhesion molecules, especially sVCAM-1, sICAM-1 and/or sE-selectin, wherein the method allows for the determining the concentration of said molecules.

In order to achieve the aforementioned objects, a method as defined in the claims is provided.

According to a first aspect of the invention, a method for detecting soluble adhesion molecules is provided, the method comprising at least the steps of:

a) Providing a fluorescence correlation spectroscopy (FCS) device comprising a loading zone for a sample;

b) Providing a sample containing at least one soluble adhesion molecule being dissolved in the sample;

c) Providing at least one fluorescence labeled antibody which is able to selectively interact to said at least one soluble adhesion molecules;

d) Allowing said at least one soluble adhesion molecule to interact with the at least one fluorescence labeled antibody in the sample;

e) Subjecting the sample to fluorescence correlation spectroscopy measurement;

f) Determining the concentration of said at least one soluble adhesion molecule in the sample.

According to one preferred embodiment of said method, the fluorescence correlation spectroscopy (FCS) device is a fluorescence cross-correlation spectroscopy (FCCS) device and the sample is subjected to a fluorescence cross-correlation spectroscopy (FCCS) measurement.

The use of FCS or FCCS for detecting, analyzing or quantifying of soluble adhesion molecules being dissolved in a sample has several advantages. For example, FCS and FCCS are very sensitive techniques and, since the described FCS and FCCS measurements are carried out in solution, the mixing times of the species are faster than in a corresponding ELISA test where the samples must interact with the antibody on a surface, and often require further complicated actuation and mixing technologies.

According to another aspect, the present invention relates to the use of a fluorescence correlation spectroscopy (FCS) device or a fluorescence cross-correlation spectroscopy (FCCS) device for detecting soluble adhesion molecules being dissolved in a sample.

The concentrations of soluble adhesion molecule like ICAM-1, VCAM-1 and/or E-selectin in the blood of children and adolescents with major risk factors for atherosclerosis (obesity, diabetes and hyper-tension) are significantly higher than in healthy subjects. Consequently, any information about the concentration of said soluble adhesion molecules can lead to earlier therapeutic intervention, and the prevention of disease progression. Therefore, according to another aspect of the present invention a method for assessing the risks or the progression of coronary artery disease (CAD) is provided, the method comprising the steps of:

a) Detecting the concentration of at least one type of soluble adhesion molecule in a blood sample by using the inventive detection method described and claimed herein;

b) Evaluating the results obtained by said detection method.

According to yet another aspect, the present invention refers to a method of diagnosing coronary artery disease (CAD) in a subject, the method comprising:

a) Detecting soluble adhesion molecules in a blood sample by using the method according to anyone of claims 1 to 9;

b) Evaluating the detection results.

The aforementioned methods can be used for assessing the risks or the progression of myocardial infarction, atherosclerosis or heart failure.

The term "coronary artery diseases" (CAD) encompasses myocardial infarction, atherosclerosis as well as heart failure.

The term "soluble adhesion molecule" as used herein refers to adhesion molecules which are soubilized in a sample and especially are solubilized in a blood sample or are able to circulate in the blood.

In the following it is referred to preferred embodiments of the present invention including the inventive detection method, and the inventive method for assessing the risks or the progression of coronary artery disease (CAD).

According to one embodiment of the present invention, the sample to be analyzed is a human blood sample. According to a further embodiment of the present invention, the blood sample prior to the spectroscopy measurement is preprocessed by the addition of a cell lysis reagent, by using a microfluidic cartridge for separating the blood cells from the serum/plasma and/or by a centrifugation step.

According to yet another embodiment of the present invention, the at least one fluorescence labeled antibody is selected from the group consisting of fluorescence labeled monoclonal human antibodies specifically or selectively binding to one soluble adhesion molecule.

The inventive method cannot only be used for detecting soluble adhesion molecules but also or alternatively for detecting cytokines, chemokines, growth factors, or even proteolyitically digested peptides. Also these substances or markers may be indicative for CAD. They include human growth factor, human nerve growth factor, platelet derived growth factor, TGFß, EGF, VEGF, TNF-a,TNF-b, FGFs, TGF-a, Epo, IGF-I, IGF-II, IL-1, IL-2, IL-6, IL-8, INF-g and CSFs as well as integrins and CAMs including VLA-1 to VLA-6, LPAM-1, LFA-1, CR3, CR4, leukointegrin, collagen, laminin, fibronectin, VCAM-1, MAdCAM-1, E-cadherin, ICAM-1, ICAM-2, ICAM-3, C3b and C4b.

According to one embodiment of the present invention, the sample is contained in a cartridge which is placed in the loading zone. The cartridge may have one or more cavities or sample volumes where one or more different samples can be analyzed (multisample cartridge). Therefore, according to another embodiment of the inventive method, the cartridge being placed in the loading zone is construed such that it can contain two, three or more different samples, i.e. has two, three or more sample volumes. Accordingly, it is possible to analyze two, three or more samples of different patient or, alternatively, different species of the soluble adhesion molecules in samples derived from the same patient.

According to another embodiment of the present invention, at least two different species of the at least one soluble adhesion molecule (e.g. soluble vascular cell adhesion molecule-1 (VCAM-1) and soluble E-selectin) are detected at the same time, i.e. simultaneously or one after another, e.g. by shifting a multisample cartridge so that one sample after the other is subjected to fluorescence correlation spectroscopy measurement. For analyzing two or more different species of the at least one soluble adhesion molecule a multisample cartridge may be used, or multiple laser sources to excite multiple distinct fluorphores, or non-linear optical methods, like two-photon fluorescence excitation, that can excite multiple fluorophores with one laser line.

According to one preferred embodiment, the soluble adhesion molecules are selected from the group consisting of intercellular adhesion molecule 1 (ICAM-1), vascular adhesion molecule 1 (VCAM-1), and selectins (E-, P- and L-), and more preferably are selected from the group consisting of vascular cell adhesion molecule-1 (VCAM-1) and E-selectin.

DETAILED DESCRIPTION OF EMBODIMENTS

Before the invention will be described in detail with respect to some of its preferred embodiments, the following general definitions are provided.

As used in this specification and in the appended claims, the singular forms of "a" and "an" also include the respective plurals unless the context clearly dictates otherwise.

It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to disclose a group, which preferably consists only of these embodiments.

Further definitions of terms will be given in the context of which the terms are used.

According to one aspect of the invention, a method for detecting soluble adhesion molecules is provided. The inventive method is especially suitable for detecting soluble adhesion molecules are selected from the group consisting of intercellular adhesion molecule 1 (ICAM-1), vascular adhesion molecule 1 (VCAM-1), and selectins (E-, P- and L-), and more preferably is selected from the group consisting of vascular cell adhesion molecule-1 (VCAM-1) and E-selectin (ELAM-1; CD62E). In a first step of the inventive detection method, a fluorescence correlation spectroscopy (FCS) device or a fluorescence cross-correlation spectroscopy (FCCS) device comprising a loading zone for a sample is provided. The loading zone of said device according to the present invention is loaded with a sample containing the at least one soluble adhesion molecule being dissolved in the sample together with at least one fluorescence labeled antibody selectively interacting or binding to said at least one soluble adhesion molecule. Suitable samples are derived from human blood and can e.g. be a blood sample or a preprocessed blood sample. The concentration or the amount of the soluble adhesion molecules being dissolved in the sample according to the present invention is measured or determined by fluorescence correlation spectroscopy (FCS) or fluorescence cross-correlation spectroscopy (FCCS). The sample which may be loaded or contained in a cartridge or sample container is placed on the loading zone of the FCS or FCCS device. By using FCS or FCCS, it is possible to differentiate between interacting or bound antibodies and unbound antibodies, thus allowing for determining the concentration of the dissolved adhesion molecules of interest which selectively interact or bind to said antibody in the sample.

Fluorescence correlation spectroscopy (FCS) and fluorescence cross-correlation spectroscopy (FCCS) are highly sensitive and versatile techniques often used for the study of dynamics and interactions of individual fluorescently labeled molecules in solution or living cells. FCS analyses minute fluorescence intensity fluctuations caused by spontaneous deviations from the mean in thermal equilibrium. The fluorescent fluctuations can be quantified in their intensity and duration by temporally autocorrelating the recorded intensity signal. The information about the processes that influence the molecular dynamics can then be extracted from the decay of the fluorescence fluctuations. The normalized auto-correlation function G($\tau$) for the fluorescence fluctuations $\delta F(t)$ of the signal F(t) is defined as the temporal average of the fluorescence signal F at time t multiplied with the signal F at a later time (t+$\tau$):

$$G(\tau) = \frac{\langle \delta F(t) \cdot \delta F(t+\tau) \rangle}{\langle F(t) \rangle^2}.$$

Fitting the recorded autocorrelation curves to a two component FCS diffusion model containing additional terms accounting for the photophysics (triplet-blinking) of the fluorescent dyes and an offset, one obtains information on the number of molecules N in the volume and the average lateral transit time $\tau_D$. Knowledge of the size of the detection volume from calibration measurements allows the determination of the setup-independent diffusion coefficient D.

Extending FCS to two colors, the excitation of two spectrally different dyes in the same detection element allows the measurement of the interactions between differently labeled molecules. Besides the two autocorrelation curves, a cross-correlation curve is acquired by correlating the two channels.

$$G_{ij}(\tau) = \frac{\langle \delta F_i(t) \cdot \delta F_j(t+\tau) \rangle}{\langle F_i(t) \rangle \langle F_j(t) \rangle}$$

Only when a dual labeled molecule translocates through the detection volume it contributes to the cross-correlation curve. Hence, the amplitude of the cross-correlation curve is directly proportional to the amount of double labeled species and allows a quantitative analysis of the interaction.

Due to the sensitivity of FCS and FCCS, simple finger prick blood volumes are sufficient for carrying out the inventive detection method. The blood sample can be used directly or, alternatively, after carrying out preprocessing steps. For example, the blood sample can be preprocessed by separating blood cells from serum. This can be achieved by, e.g., the addition of a cell lysis reagent, by using a microfluidic cartridge for cell/serum separation or a centrifugation step prior to adding the serum to the device. More specifically, blood can either be placed in an anticoagulant coated sample tube and centrifuged or lysed to yield the sample. Alternately, the blood processing could be automated in a small microfluidic chip that separates serum from cells. The fluidic device could also employ mixing of a lysis agent to yield the final sample. There are alternative preprocessing steps which could be used and are well-known to the skilled person. After the mixing of the serum or the sample with fluorescence labeled antibodies, it is possible to dynamically monitor the levels of soluble adhesion molecules. The addition of the fluorescent antibody (or antibodies) can be at various points, either directly to the blood, serum or lysed sample, or mixed with the sample within the microfluidic chip or detection volume holder. The mixing of sample and antibody in solution is more rapid than that on a surface. Magnetic or electro-actuated mixing, or micro-fluidic mixing can improve the speed of the mixing or interaction within the assay.

In one embodiment of the invention, the serum/blood sample can be mixed with the antibodies prior to addition to the sample container or cartridge. In another embodiment the blood sample and antibody or antibodies can be simultaneously introduced and mixed in a microfluidic type cartridge. The antibody/serum cocktail is preferably incubated for 1-30 minutes after-which the sample is illuminated with the laser light.

As antibodies bind to the target (soluble adhesion molecule) and diffuse through the excitation volume (i.e. the laser focus) they give off a burst of fluorescence. The total light detected from a single color channel corresponds to the number of fluorophores present in the excitation volume at a given time point. Over time there is a slight fluctuation in this signal since at specific times there can be slightly more or slightly less fluorescent particles in the observation volume. Fitting these time resolved fluorescence fluctuations with an autocorrelation function yields the autocorrelation curve. The curve contains two major types of data, namely, the average number of particles per excitation volume, i.e. the concentration of molecular species, as represented by the inverse of the Y-intercept of the curve, or the G(o) value (that is the G(o) is inversely proportional to the concentration), and, secondly the diffusion coefficient of the diffusing molecular species, represented by the length of the decay of the autocorrelation curve. Since the diffusion coefficient of a molecular species is proportional to the hydrodynamic radius (and molecular weight) of that species, the unbound antibodies can be differentiated from the soluble adhesion molecules interacting with or being bound to the antibodies. In the event that the bound and unbound antibodies are not resolvable by FCS methods (which may be the case if the change in molecular weight between bound and unbound states is less than a factor of two), the sample may be subjected to fluorescence cross-correlation spectroscopy measurement (FCCS). In FCCS, like with FCS, the time resolved fluorescence intensity fluctuations, of this time two distinct fluorescence labeled species, is monitored and cross-correlated. The resulting cross correlation curve looks similar to the autocorrelation curve, only that the number of diffusing species is now directly proportional to the Y-intercept, G(x) value. Again the decay of the curve is indicative of the diffusion time of the fluorescence species.

Therefore, according to one preferred embodiment of the inventive detection method, the fluorescence correlation spectroscopy (FCS) device is a fluorescence cross-correlation spectroscopy (FCCS) device and the sample is subjected to a fluorescence cross-correlation spectroscopy (FCCS) measurement. The FCCS method may be preferred, since it only requires one correlation fit, of the dual labeled diffusing species, and hence unbound antibody is not observed and must not be corrected for.

If fluorescence correlation spectroscopy (FCS) is used as the detection method, modified equations can be used to fit a single curve and obtain both, the concentrations of bound and unbound species. This is due to the fact that one knows the molecular weight and may also know the amount of the fluorescent species (i.e. antibody) used for the interacting or binding reaction (it is the soluble marker that is of unknown concentration only). These are called multi-component fits and are standard in biophysical studies using FCS.

In another preferred embodiment of the present invention, the FCS or FCCS device only requires a highly defined excitation volume (i.e. a focused laser into a sample volume) as well as the proper fluorescence detection filters (which are also required for ELISA methods). Furthermore, the basic device useful according to the present invention would contain a simple illumination source, which preferably is a laser, being focused into the sample solution in the loading zone. The sample solution can be contained in a sample cartridge or container, which may be disposable. The sample or cartridge is put into a loading zone or specific sample holder slide and is illuminated with the appropriate wavelength of excitation light. By using a highly focused laser, only fluorophores in the very small detection volume of the laser focus are excited and fluoresce. The emitted fluorescence light is then collected with e.g. a photomultiplier (PMT) or an avalanche diode (APD) and analyzed for time-fluorescence intensity fluctuations.

In a preferred embodiment of the invention, the FCS or FCCS device would be a three color capable device targeting sVCAM-1, sICAM-1 and sE-selectin with distinctly labeled antibodies. Such antibodies are well-known to the skilled person and are commercially available. For example, human VCAM-1 (CD 106) antibodies are commercially available by BioLegend (Catalogue # 305809, 305810, 305803, 305806, etc.), human ICAM-1 (CD 54) antibodies are commercially available by Bio-Legend (Catalogue # 313108, 322705) and by e.g. AbDSerotec ( Product Code MCA2507) and human E-selectin (CD62E) antibodies are commercially available by BioLegend (Catalogue # 322603, 322604, 322602 etc.) and by e.g. AbDSerotec ( Product Code MCA1969). The antibodies can be fluorescence labeled by the methods known to the art. Typical fluorescence label molecules comprise fluorescein isothiocyanate (FITC), R-phycoerythrin (R-PE), and R-PE:cyanine-5 (PE:Cy5) fluorochromes, fluorescein, 5,6-carboxymethyl fluorescein, (5-carboxyfluorescein-N-hydroxysuccinimide ester), mtrobenz-2-oxa-1,3-diazol-4-yl (NBD), coumarin, dansyl chloride, and rhodamine (5,6-tetramethyl rhodamine). These can be obtained from a variety of commercial sources, including Molecular Probes, Eugene, OR and Research Organics. The fluorescent antibodies function as targeted labels for the respective adhesion molecules.

In another preferred embodiment of the invention, the FCS or FCCS device would be a two color capable device targeting sVCAM-1 and sE-selectin with distinctly labeled antibodies.

In another preferred embodiment of the inventive method, a sample container or cartridge with two, three or more simultaneous or parallel detection volume chambers can be used. In another embodiment, separate designated sample cartridges are utilized. Since one only requires 5-60 seconds of acquisition time to obtain a correlation curve, and the amount of sample produced by a finger prick is sufficient for multiple analysis, it is possible to analyse several tailored sample cartridges, each designed for the specific detection of one molecular species in the sample. According to another embodiment, a panel of disposable cartridges designated for various stages of CAD is used. In yet another embodiment, a multisample holding cartridge is utilized and fixed in the loading zone of the device, and the laser focus or the cartridge itself is displaced to analyse each individual species within each individual chamber in the device.

According to another embodiment of the present invention, a laser light focus is not required. The definition and illumination of an excitation volume can be usually most easily achieved with a laser focus. However, LED or other lamp illuminations may also be suitable if the illumination volume can be defined and controlled and the subsequent light emitted from the volume collected. In addition, the use of non-linear optics (i.e. multi-photon excitation) allows for the excitation of multiple fluorophores with a single laser line. While these optics may be more complex than a single one photon illumination source, for multi-color detection, the device may require less components (i.e. filters or lasers of different wavelength) making multi-photon excitation a feasible option for use in the present invention.

According to another aspect, the present invention relates to the use of a fluorescence correlation spectroscopy (FCS) device or a fluorescence cross-correlation spectroscopy (FCCS) device for detecting soluble adhesion molecules being dissolved in a sample. Suitable fluorescence correlation spectroscopy (FCS) devices and fluorescence cross-correlation spectroscopy (FCCS) devices are described above. According to one preferred embodiment, the soluble adhesion molecules are selected from the group consisting of intercellular adhesion molecule 1 (ICAM-1), vascular adhesion molecule 1(VCAM-1), and selectins (E-, P- and L-), and more preferably is selected from the group consisting of vascular cell adhesion molecule-1 (VCAM-1) and E-selectin.

The concentrations of soluble adhesion molecule like ICAM-1, VCAM-1 and/or E-selectin and especially of VCAM-1 and E-selectin in the blood of children and adolescents with major risk factors for atherosclerosis (obesity, diabetes and hyper-tension) are significantly higher than in healthy subjects. Therefore, according to another aspect of the present invention a method for assessing the risks or the progression of coronary artery disease (CAD) is provided, the method comprising the steps of:

a) Detecting the concentration of at least one type of soluble adhesion molecule in a blood sample by using the inventive detection method described and claimed herein;
b) Evaluating the results obtained by said detection method.

Even minute changes in the levels of adhesion molecules like e.g. E-selectin may be indicative for changes in the progression of CAD, even in adults with more progressed disease, and, thus, may provide novel windows for prognostics, therapy decisions and alterations. Such minute changes are only resolvable by ultra-sensitive spectroscopic techniques, such as FCS and FCCS. For example, the inventive method allows for detecting an increasing risk for CAD over the time in view of increasing level or concentration of soluble adhesion molecule like ICAM-1, VCAM-1 and/or E-selectin and especially of VCAM-1 and E-selectin in the blood of a human subject or patient. Furthermore, the inventive method allows for assessing the success or failure of a therapy or the effectiveness of medicaments which should reduce the risk for CAD by monitoring the level or concentration of soluble adhesion molecule like ICAM-1, VCAM-1 and/or E-selectin and especially of VCAM-1 and E-selectin.

By using the detection method of the present invention, the concentration of soluble adhesion molecules can be monitored in CAD patients. The relatively simple optical set up needed for carrying out the inventive detection method or the FCS or FCCS measurement, makes routine monitoring of these parameters very simple. Accordingly, if a sharp increase in the concentration of one or more soluble adhesion molecules is observed over the course of days or weeks, it may be indicative of the need for therapeutic intervention. Like-wise, if a CAD patient begins a new medication, and this correlates to a decrease in the levels of sVCAM-1, sICAM-1 and/or sE-selectin, one can continue the therapy, or lower the dose to the maximize the therapeutic effect whilst minimizing side-effects. In addition, slight dynamics in the levels of soluble adhesion molecules in patients presenting symptoms in the Emergency Room may add a novel level of stratification in evaluating which patients can be safely discharged and which patients require immediate admittance, treatment and monitoring.

Beside the foregoing uses and applications, the inventive detection method can also be used as a clinical research tool to validate various blood based biomarkers, for various diseases. For example, investigated sample need not be blood or serum, making all kinds of tissue extracts and fluids possible, such as cell lysates, biopsy lysates, urine, stool or breast fluid. The instrument can also be utilized as a basing life science research tool examining diffusion of biomolecules in aqueous solutions.

Further possible applications for the present invention relate to prognosis and risk stratification, as well as therapy evaluation for colorectal cancer, breast cancer, prostate cancer, lung cancer, ovarian cancer, urinary bladder and kidney cancers, leukemia, lymphoma as well as. Furthermore, any disease that levels an impact on circulating biomolecules could potentially be investigated with such a tool, provided the proper antibodies or targeting molecules exist. This includes infectious diseases, drug testing, auto-immune disorders, genetic and inheritable diseases.

In the following, the present invention is further illustrated by referring to embodiment examples:

In one embodiment example of the invention, a finger prick blood is deposited in a small anticoagulant coated tube and centrifuged for 10-30 min. The blood cells will sediment and the top fluid layer contains the serum with the soluble adhesion molecules. According to another embodiment example, the blood prick sample is centrifuged through a density gradient (such as buffy coat, percoll or ficoll) again yielding sedimented blood cells and serum on the top layer. In yet another embodiment example, the blood sample is treated with a lysis reagent (detergent, or ammonium chloride, etc) to lyse either all blood cells or just red blood cells, and the resulting solution containing serum, lysis solution and lysed cells is utilized as the sample. In still another embodiment example, the blood prick is directly deposited on a cartridge, where microfluidic channels are utilized to separate serum from blood cells.

As a next step, a fluorescence labeled monoclonal human antibody specifically binding to VCAM-1 is added to the sample. After an incubation time of 20 to 30 minutes, the sample is subjected to FCS or FCCS measurement. Another sample from the same patient may be preprocessed the same way. After incubating the sample with a fluorescence labeled monoclonal human antibody specifically binding to E-selectin, the sample is subjected to FCS or FCCS measurement. The obtained values or concentrations of VCAM-1 and E-selectins are then evaluated in order to assess the risks of CAD and, if necessary initiate a therapy, modify an ongoing therapy or maintaining the therapy.

## Claims

1. A method for detecting soluble adhesion molecules, the method comprising at least the steps of:

   a) Providing a fluorescence correlation spectroscopy (FCS) device comprising a loading zone for a sample;
   b) Providing a sample containing at least one soluble adhesion molecule being dissolved in the sample;
   c) Providing at least one fluorescence labeled antibody which is able to selectively interact to said at least one soluble adhesion molecule;
   d) Allowing said at least one soluble adhesion molecule to interact with the at least one fluorescence labeled antibody in the sample;
   e) Subjecting the sample to fluorescence correlation spectroscopy measurement;
   f) Determining the concentration of said at least one soluble adhesion molecule in the sample.

2. Method according to claim 1, wherein the fluorescence correlation spectroscopy (FCS) device is a fluorescence cross-correlation spectroscopy (FCCS) device and the sample in step d) is subjected to fluorescence cross-correlation spectroscopy measurement.

3. Method according to claims 1 or 2, wherein the soluble adhesion molecules are selected from the group consisting of intercellular adhesion molecule 1 (ICAM-1), vascular adhesion molecule 1(VCAM-1), and selectins (E-, P- and L-), and more preferably are selected from the group consisting of vascular cell adhesion molecule-1 (VCAM-1) and E-selectin.

4. Method according to any of the foregoing claims, wherein the sample is a human blood sample.

5. Method according to claim 4, wherein the blood sample prior to the spectroscopy measurement is preprocessed by the addition of a cell lysis reagent, by using a microfluidic cartridge for separating the blood cells from the serum and/or by a centrifugation step.

6. Method according to any of the foregoing claims, wherein the at least one fluorescence labeled antibody is selected form the group consisting of fluorescence labeled monoclonal human antibodies specifically binding to one soluble adhesion molecule.

7. Method according to any of the foregoing claims, wherein the sample is contained in a cartridge which is placed in the loading zone.

8. Method according to claim 7, wherein the cartridge is construed such that it can contain two, three or more different samples.

9. Method according to any of the foregoing claims, wherein at least two different species of the at least one soluble adhesion molecule are detected at the same time or one after another.

10. Use of a fluorescence correlation spectroscopy (FCS) device for detecting at least one soluble adhesion molecule.

11. Use according to claim 10, wherein the fluorescence correlation spectroscopy (FCS) device is a fluorescence cross-correlation spectroscopy (FCCS) device.

12. Method according to claims 10 or 11, wherein the at least one soluble adhesion molecule is selected from the group consisting of soluble intercellular adhesion molecule 1 (ICAM-1), soluble vascular adhesion molecule 1 (VCAM-1), and soluble selectins (E-, P- and L-), and more preferably are selected from the group consisting of soluble vascular cell adhesion molecule-1 (VCAM-1) and soluble E-selectin.

13. Method for assessing the risks or the progression of coronary artery disease (CAD) in a subject comprising the steps of:

   a) Detecting soluble adhesion molecules in a blood sample by using the method according to anyone of claims 1 to 9;
   b) Evaluating the detection results.

14. Method of diagnosing coronary artery disease (CAD) in a subject, the method comprising:

   c) Detecting soluble adhesion molecules in a blood sample by using the method according to anyone of claims 1 to 9;
   d) Evaluating the detection results.

15. Method according to claims 13 or 14, wherein the method is used for assessing the risks or the progression of myocardial infarction, atherosclerosis or heart failure.

Europäisches
Patentamt

European
Patent Office

Office européen
des brevets

# EUROPEAN SEARCH REPORT

Application Number

EP 08 16 4089

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JOHNSON COLIN P ET AL: "Direct evidence that neural cell adhesion molecule (NCAM) polysialylation increases intermembrane repulsion and abrogates adhesion" 7 January 2005 (2005-01-07), JOURNAL OF BIOLOGICAL CHEMISTRY, VOL. 280, NR. 1, PAGE(S) 137-145 , XP002504132 ISSN: 0021-9258 * page 138, left-hand column, paragraph 4 * * page 139, right-hand column, paragraph 6 - page 140, right-hand column, paragraph 3 * ----- | 1-9, 13-15 | INV. G01N33/68 |
| Y | US 2003/124599 A1 (CHEN SHIPING [US] ET AL) 3 July 2003 (2003-07-03) * paragraphs [0015], [0016], [0061], [0065], [0068], [0081] * ----- | 1-9, 13-15 | |
| Y | WO 03/023573 A (BIOSEEK INC [US]; BERG ELLEN L [US]; BUTCHER EUGENE C [US]; MELROSE JE) 20 March 2003 (2003-03-20) * page 183, right-hand column, paragraph 3 * * page 184, left-hand column, paragraph 4 - page 188, right-hand column, paragraph 1 * ----- | 1-9, 13-15 | TECHNICAL FIELDS SEARCHED (IPC)  G01N |
| Y | WO 2008/073634 A (UNIV MICHIGAN [US]; EITZMAN DANIEL T [US]; BODARY PETER F [US]; HOMEIS) 19 June 2008 (2008-06-19) * claims 1,5-7,9 * ----- | 13-15 | |
| Y | EP 1 714 661 A (BLOOD RES CENTER [US]) 25 October 2006 (2006-10-25) * paragraph [0035] * * claims 21-25 * ----- | 13-15 | |
| | -/-- | | |

~~The present search report has been drawn up for all claims~~

| Place of search | Date of completion of the search | Examiner |
|---|---|---|
| The Hague | 17 November 2008 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 16 4089

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RUAN ET AL: "Applications of dual-color fluorescence cross-correlation spectroscopy in antibody binding studies" ANALYTICAL BIOCHEMISTRY, ACADEMIC PRESS INC. NEW YORK, vol. 374, no. 1, 4 December 2007 (2007-12-04), pages 182-195, XP022437949 ISSN: 0003-2697 * the whole document * | 1-15 | |
| A | US 2005/148023 A1 (THADHANI RAVI I [US] ET AL THADHANI RAVI I [US] ET AL) 7 July 2005 (2005-07-07) * the whole document * | 1-15 | |

TECHNICAL FIELDS
SEARCHED      (IPC)

~~The present search report has been drawn up for all claims~~

| Place of search | Date of completion of the search | Examiner |
|---|---|---|
| The Hague | 17 November 2008 | Chrétien, Eva Maria |

Europäisches
Patentamt

European
Patent Office

Office européen
des brevets

**Application Number**

EP 08 16 4089

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9, 13-15

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 08 16 4089

The Search Division considers that the present European patentapplication does not comply with the requirements of unity of invention and relates to severalinventions or groups of inventions, namely:

1. claims: 1-9,13-15

   Methods for detecting soluble adhesion molecules via FCS using specific monoclonal antibodies
   ---

2. claims: 10-12

   Using FCS for detecting soluble adhesion molecules
   ---

## ANNEX TO THE EUROPEAN SEARCH REPORT
## ON EUROPEAN PATENT APPLICATION NO.

EP 08 16 4089

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2008

| Patent document cited in search report | | Publication date | Patent family member(s) | | Publication date |
|---|---|---|---|---|---|
| US 2003124599 | A1 | 03-07-2003 | NONE | | |
| WO 03023573 | A | 20-03-2003 | EP | 1436617 A2 | 14-07-2004 |
| WO 2008073634 | A | 19-06-2008 | NONE | | |
| EP 1714661 | A | 25-10-2006 | NONE | | |
| US 2005148023 | A1 | 07-07-2005 | NONE | | |

EPO FORM P0459

For more details about this annex : see Official Journal of the European Patent Office, No. 12/82